# NEW EUROPEAN PATENT SPECIFICATION

(11) **EP 1 932 555 B2**
(45) Date of publication and mention of the opposition decision: **08.06.2016**
(45) Mention of the grant of the patent: 02.06.2010
(21) Application number: 07123002.3
(22) Date of filing: 12.12.2007
(51) Int. Cl.: A61M 5/142, A61M 5/145, A61M 5/172, G01D 5/244

(54) **Optical detection of medical pump rotor position**
Optische Erfassung der Position des Rotors einer medizinischen Pumpe
Détection optique de position de rotor de pompe médicale

(30) Priority: 15.12.2006 US 611693
(43) Date of publication of application: 18.06.2008
(73) Proprietor: Covidien AG, 8212 Neuhausen am Rheinfall (CH)
(72) Inventor: Lewis, Thomas G., O`Fallon, IL 62269 (US); Davis, Mark A., O`Fallon, IL 63366 (US)
(74) Representative: Körber, Martin Hans

(56) References cited:
- US-A- 3 673 476
- US-A- 3 982 162
- US-A- 4 454 763
- US-A- 4 806 751
- US-A- 5 531 680
- US-A1- 2004 064 088
- US-A1- 2005 267 439
- US-B1- 6 234 992
- US-B1- 6 299 600
- G. DROSDOWSKI, PROF. DR. DR. H.C. ET AL.: 'Duden Deutsches Universalwörterbuch', vol. 3, 1996, DUDEN VERLAG, MANNHEIM, LEIPZIG, WIEN, ZÜRICH page 932
- M. WERMKE, DR. ET AL.: 'Duden Das Bedeutungswörterbuch', vol. 10, 2002, DUDENVERLAG, MANNHEIM, LEIPZIG, WIEN, ZÜRICH page 792
- "Rotor", ¬Online| 24 November 2006, Retrieved from the Internet: URL:http://de.wikipedia.org/w/index.php? title-Rotor & oldid=24251334 ¬retrieved on 2011-02-23|
- "Hatching", ¬Online| 12 December 2006, Retrieved from the Internet: URL:http://en, wikipedia.org/w/index.php? title=Hatching&oldid=93712614 ¬retrieved on 2011-02-23|
- Merriam Webster: "Scatter", ¬Online| Retrieved from the Internet: URL:http://www.merriam-webster.com/dictiona ry/scatter ¬retrieved on 2012-03-21|
- Merriam Webster: "Absorb", ¬Online| Retrieved from the Internet: http://www.merriam-webster.com/dictionary/a bsorbing ¬retrieved on 2012-03-21|
- Merriam Webster: "Rotor", ¬Online| Retrieved from the Internet: URL:http://www.merriam-webster:com/dictiona ry/rotor ¬retrieved on 2012-03-22|
- Merriam Webster: "Motor", ¬Online| Retrieved from the Internet: URL:http://www.merriam-webster.com/dictiona ry/motor ¬retrieved on 2012-03-22|
- 'Non-reflective glass- what is the difference' NEWSLETTER 2 29 October 2014, pages 1 - 4
- Frametoday, retrieved from the Internet: URL:http://www.frametoday.com.au/picture-fr ame-glass ¬retrieved on 2014-10-29|
- "TFT Central: Panel Coating", ¬Online| retrieved from the Internet: URL:http://www. tftcentral.co.uk/articles/panel-coating.htm ¬retrieved on 2014-10-29|

## Description

### BACKGROUND

Detecting the position and rotation of a pump rotorof a pumping apparatus (e.g. a rotary style peristaltic pump) allows the pumping apparatus to determine a rate of fluid delivery as well as some error conditions of the pumping apparatus. Typically, an electric motor drives the pump rotor such that pump rotor rotational speed and position can be estimated by monitoring a current and/or a voltage of the electric motor. However, some pumping applications, such as pumps which deliver medical fluids to a patient, require greater accuracy. One approach is to position magnets in a surface of the pump rotor and detect rotation of the pump rotor via a nearby Hall Effect sensor. This approach requires relatively expensive magnets and Hall Effect sensors and is necessarily adversely affected by other magnetic fields. Additionally, Hall Effect sensors produce partial sinusoid detection signals, the transitions times of which limit the amount of time that the detection signals spend at higher magnitudes, thus increasing the likelihood of detection signal inaccuracies. For example, if the detection signal is being digitally sampled and compared to a threshold, the sample may miss the peak of the sinusoid signal causing the system to miss detecting a magnet passing by the sensor. This effect would become more likely for relatively low sample rates and relatively high rotational speeds.

US 6,234,992 B1 discloses a medical pumping apparatus comprising a pump rotor, a motor for rotating the pump rotor, an emitter for emitting electromagnetic radiation and a detector for receiving electromagnetic radiation providing a detection signal signal indicative of the received electromagnetic radiation for monitoring rotation of the pump rotor, whereby the electromagnetic radiation received by the detector is radiation directly passing from the emitter to the detector and monitoring of the rotation of the pump rotor is based on the blocking of a light path from the emitter to the detector by the pump rotor.

US 2004/0064088 A1 discloses a medical pumping apparatus wherein a motor driven lead screw performs a longitudinal movement upon rotation, the longitudinal movement actuating a syringe mechanism. An encoder disk is provided co-rotating with the lead screw having radially spaced light reflective indicia thereon. A light emitter and a light detector are directed at the encoder disk. The light emitter/detector is connected to a processor which is programmed to determine the amount of rotation of the lead screw.

The document US 5,531,680 shows the features of the preamble of the independent claims.

### SUMMARY

A medical pump embodying aspects of the invention provides a more cost effective monitoring approach that is not adversely affected by magnetic fields. A medical pumping apparatus according to the present invention is defined in claim 1 and a pump rotor according to the present invention is defined in claim 6.

### BRIEF DESCRIPTION OF THE DRAWINGS

FIG. 1 is a perspective view of a pumping apparatus.
FIG. 2 is perspective, exploded, and partially schematic view of a pumping apparatus illustrating one embodiment having part of a housing of the pumping apparatus cut away wherein an emitter detector pair is mounted in the housing.
FIG. 3 is a perspective, exploded, and partially schematic view of a pumping apparatus illustrating one embodiment having part of a housing of the pumping apparatus cut away wherein an emitter-detector pair is mounted behind a transmissive window of the housing.
FIG. 4 is a partially exploded view of a pump rotor.
FIG. 5 is a partial perspective view of a pumping apparatus illustrating a disk mounted on a shaft operatively connected to a motor of the pumping apparatus and monitored by an emitter detector pair.
FIG. 6A is a partial side view of a pumping apparatus illustrating a disk on a shaft of a motor of the pumping apparatus monitored by an emitter detector pair.
FIG. 6B is a partial end view of the pumping apparatus of FIG. 5A illustrating the disk and emitter detector pair.
FIG. 7 is a perspective view of a pumping apparatus illustrating a disk with notches mounted on a shaft operatively connected to a motor of the pumping apparatus and monitored by an emitter detector pair.

Corresponding reference characters indicate corresponding parts throughout the drawings.

### DETAILED DESCRIPTION

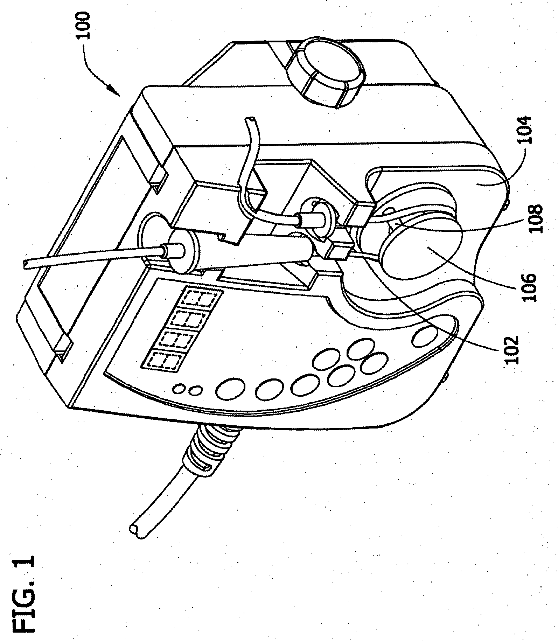

Referring to FIG. 1, a medical pumping apparatus is generally designated 100. A pump set 102 which supplies fluid from a reservoir (not shown) to a patient (not shown) is loaded on a housing 104 of the pumping apparatus 100 such that the pump set 102 is in contact with a pump rotor 106 of the pumping apparatus 100. When the pumping apparatus 100 rotates the pump rotor 106, rollers 108 occlude a portion of a tube of the pump set 102 and force the fluid from the reservoir to the patient by a peristaltic action. The pumping apparatus 100 controls rotation of pump rotor 106 to control the rate and volume of fluid delivery. Aspects of the invention permit monitoring the position and rotation of pump rotor 106 with greater accuracy and greater cost-effectiveness than conventional approaches. Embodiments of the invention achieve greater accuracy by employing sensors that are relatively unaffected by magnetic fields and produce square wave detection signals with relatively fast transition times.

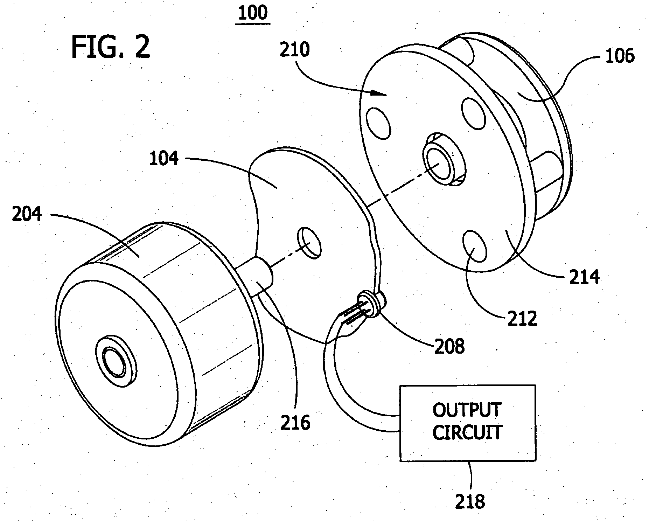

Referring to FIG. 2, the pumping apparatus 100 of FIG. 1 is shown partially exploded with a portion of the housing 104 cut away according to a first embodiment of the invention. The pumping apparatus 100 includes the housing 104, a motor 204, the pump rotor 106, and an electromagnetic radiation emitter-detector pair 208. Only the front panel of the housing 104 is shown so that the positions of the motor 204 and the emitter-detector pair 208 relative to the pump rotor 106 are visible. The electromagnetic radiation emitter-detector pair 208 emits electromagnetic radiation having a predetermined wavelength (e.g., infrared radiation or green light), and receives electromagnetic radiation including electromagnetic radiation having the predetermined wavelength. For simplicity, the electromagnetic radiation emitted by the emitter-detector pair 208 will be referred to herein as infrared radiation (IR). However, it is contemplated that the emitter-detector pair 208 may operate (i.e., emit and detect) at any wavelength and/or at multiple wavelengths without deviating from the scope of the invention.

A surface (generally designated 210) of the pump rotor 106 has a reflective portion 212 and a non-reflective portion 214. The reflective portion 212 reflects IR, and the non-reflective portion 214 absorbs, scatters, diffuses, or otherwise disperses IR such that it reflects no IR, or significantly less IR than the reflective portion 212. A shaft 216 of motor 204 passes through the housing 104 and is attached to (i.e., glued, friction fitted, fastened, , or otherwise engaged by) the pump rotor 106 at the surface 210. The shaft 216 supports the pump rotor 106 and defines its axis of rotation. When the pumping apparatus 100 supplies power to the motor 204, the motor 204 provides rotational force to the shaft 216, causing the pump rotor 106 to rotate. The emitter-detector pair 208 is mounted in the housing 104 and positioned such that as the pump rotor 106 rotates, the IR emitted by the emitter-detector pair 208 sequentially strikes the reflective portion 212 and then the non-reflective portion 214 and so forth. It is contemplated that the emitter-detector pair 208 may be mounted back from the front panel of the housing 104 and positioned to interact with the surface 210 of the pump rotor 106 through a hole, slot, or other opening in the front panel of the housing 104 without deviating from the scope of the invention.

In operation, when the non-reflective portion 214 of the pump rotor 106 is rotated through the I R emitted by the emitter-detector pair 208, the emitter-detector pair 208 receives relatively little, or no, reflected IR. Instead, the IR is scattered, absorbed, diffused, dispersed, or the like as described above. When the reflective portion 212 of the surface 210 of the pump rotor 106 rotates through the IR emitted by the emitter of emitter-detector pair 208, the detector of emitter-detector pair 208 receives a significant amount of IR (e.g., substantially equal to the amount of IR it is emitting). In one embodiment, the IR emitter-detector pair 208 generates a detection signal proportional to the amount of IR it receives. An output circuit 218, which may be part of the controller of pumping apparatus 100, receives the detection signal and compares it to a threshold. In turn, the output circuit 218 generates an output signal indicating whether the detector 208 is receiving a predetermined amount of I R reflected by the pump rotor 106 (i.e., whether the detection signal is in excess of the threshold). The threshold is a constant that is determined as a function of the construction and configuration of the pumping apparatus 100. Those skilled in the art will appreciate that the threshold is advantageously set at a level such that IR interference or noise does not cause the detection signal to exceed the threshold while still consistently indicating when the reflective portion 212 of the surface 210 of the pump rotor 106 is passing through the IR emitted by the IR emitter-detector pair 208. Thus, by monitoring the output signal for changes, a controller of the pumping apparatus 100 can determine whether the pump rotor 106 is rotating, the speed of the rotation, and the number of revolutions of the pump rotor 106 in a given period of time. Additionally, depending on the number and position of reflective 212 and non-reflective 214 portions of the surface 210 of the pump rotor 106, the output signal can be used to approximate an angular position of the pump rotor 106. The rotational speed, number of revolutions, and angular position of the pump rotor 106 may be used in any number of ways including, for example, determining a volume and rate of fluid pumped by the pumping apparatus 100.

In the pump rotor 106 of FIG. 2, the surface 210 of the pump rotor 106 has three reflective portions 212. The reflective portions 212 are spaced equal distances from the shaft 216, and are spaced equal distances from each other. The rest of the surface 210 is non-reflective such that the reflective portions 212 are separated by a single non-reflective portion 214. A controller of the pumping apparatus 100 can thus determine the angular position of the pump rotor 106 to within 120 degrees. This example is for illustration only; the pump rotor 106 may have any number of alternating reflective 212 and non-reflective 214 portions as is practical given the size of pump rotor 106.

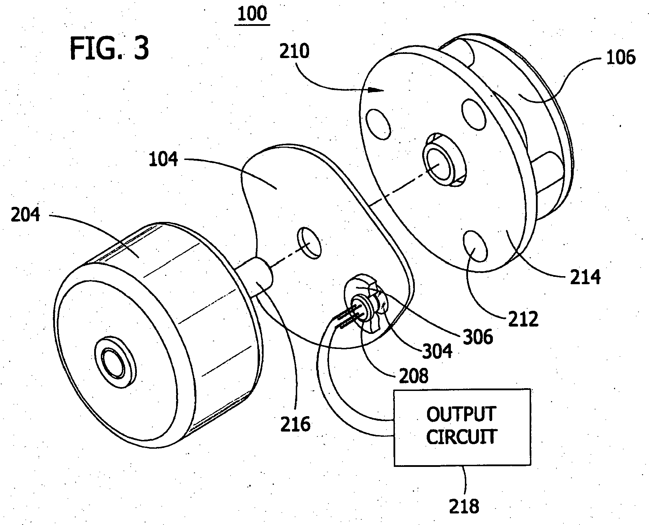

Referring now to FIG. 3, another embodiment of the pumping apparatus 100 is shown. The pumping apparatus 100 includes the motor 204, IR emitter-detector pair 208, output circuit 218, pump rotor 106, housing 104, and a transmissive window 304. The IR emitter-detector pair 208 is positioned within the housing 104 relative to the window 304 by a mount 306. In the illustrated embodiment, the mount 306 spaces the emitter-detector pair 208 back from the front panel of the housing 104. The IR emitter-detector pair 208 is positioned so that IR emitted by the emitter-detector pair 208 passes through the I R transmissive window 304 and sequentially interacts with the reflective portions 212 and the non-reflective portion 214 of the surface 210 of the pump rotor 106 as the pump rotor 106 rotates. The transmissive window 304 may be made of any material such as glass or plastic that permits IR to pass through.

The pump rotor 106 may be formed by various methods. In one embodiment, the pump rotor 106 may be injection molded using a mold that has smooth portions which correspond to the reflective portions 212, and a rough portion corresponding to the non-reflective portion 214. Alternatively, the pump rotor 106 may be machined from a block of material (e.g., plastic or aluminum) such that the pump rotor 106 has smooth portions corresponding to the reflective portions 212, and a rough (e.g., marred, hatched, scratched, or otherwise has scattering or absorptive properties relative to the electromagnetic radiation) portion corresponding to the non-reflective portion 214.

Alternatively, the pump rotor 106 may be formed (e.g., molded or machined) such that the entire surface 210 of the pump rotor 106 is reflective with respect to IR. The non-reflective portion 214 would then be added by machining (e.g., scratching or marring) the surface 210 of the pump rotor 106 to generate the non-reflective portion 214 that bounds the reflective portions 212.

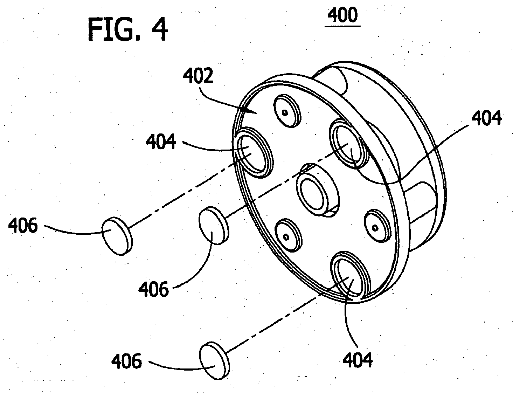

FIG. 4 illustrates an exemplary pump rotor generally designated 400 that may be used in the pumping apparatus 100 of FIGS. 1-3 in place of the pump rotor 106. The pump rotor 400 is formed such that a surface (generally designated 402) of the pump rotor 400 is non-reflective. The surface 402 has slots, recesses, or receptacles 404, for example, the receptacles required for magnets in the prior art method of pump rotor rotation detection using magnets and Hall Effect sensors. IR reflective material 406 is affixed to the pump rotor 400 in each of the receptacles 404. Alternatively, the pump rotor 400 may be formed such that the surface 402 is reflective and the material 406 affixed to the pump rotor 400 in the receptacles 404 is non-reflective without deviating from the scope of the invention. Additionally, the surface 402 may be flat and the reflective material 406 may be affixed to the surface 402 such that the receptacles 404 are not required.

It is contemplated that the reflective and non-reflective portions may be interchanged without deviating from the scope of the invention. It is also contemplated that the output circuit 218 may generate an output signal indicating that the non-reflective portion 214 of the pump rotor is passing through the IR emitted by the emitter 208 as opposed to indicating that the reflective portion 212 of the pump rotor 106 is passing through the IR emitted by the emitter 208, and that the output circuit 208 may be integral with the IR emitter-detector pair 208 or the controller of the medical pumping apparatus 100. Additionally, there may be any number of reflective and non-reflective portions of the pump rotor, and the reflective and non-reflective portions need not be located on the end of the pump rotor (e.g., they may spaced about the circumference of the pump rotor and the IR emitter-detector pair 208 positioned appropriately to detect the reflective and non-reflective portions). It is also contemplated that the reflective and non-reflective portions may not be evenly spaced from each other as illustrated , and that the emitter and detector pair 208 may operate at a frequency or wavelength other than IR. It is also contemplated that the surface of the pump rotor 106 or 400 having a reflective and non-reflective portion may be other than flat without deviating from the scope of the invention.

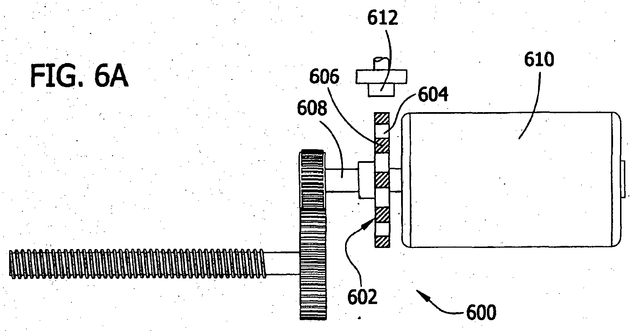

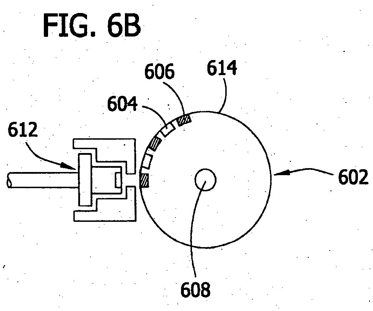

Referring now to FIGS. 6A and 6B, a portion of another exemplary pumping apparatus, generally designated 600, is shown. Here , a disk 602 having reflective 604 and non-reflective 606 portions on a peripheral surface 614 of the disk 602 is mounted on a shaft 608 of a motor 610 of the pumping apparatus. As the motor 610 rotates the shaft 608, the disk 602 is also rotated. The reflective 604 and non-reflective 606 portions sequentially pass in front of an emitter detector pair 612 mounted to monitor the periphery of the disk 602. That is, the emitter detector pair 612 is mounted in a plane defined by the disk 602 and directed at the disk 602. This example (shown in FIGS. 6A and 6B) monitors rotation of a pumping mechanism of the pumping apparatus 600 as described above for driving, for example, a worm gear shaft of a syringe pump.

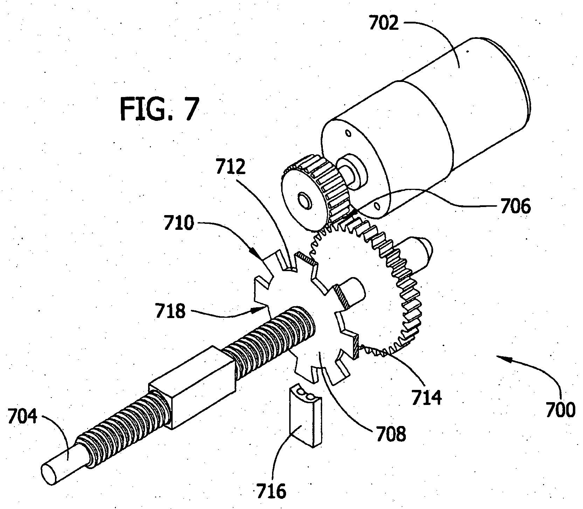

Referring now to FIG. 7, a portion of a pumping apparatus, generally designated 700, having a worm gear driven syringe pumping mechanism is shown. The pumping mechanism is driven by a motor 702, which transfers force to a worm gear shaft 704 via a gear set 706. A disk 708 affixed to the worm gear shaft 704 has an outer peripheral surface 710 with non-reflective portions 714, and an inner peripheral surface 718 with reflective portions 712. Thus, the disk 708 appears to have notches where the outer surface 710 transitions to the inner surface 718. An emitter detector pair 716 is positioned in a plane defined by the disk 708 and directed toward the disk 708, such that radiation emitted by the emitter detector pair 716 interacts with the inner and outer peripheral surfaces of the disk 708. The emitter detector pair 716 is focused on the inner peripheral surface 718 such that the emitter detector pair 716 receives substantially more reflected radiation from the reflective portions 712 than the non-reflective portions 714 because the non-reflective portions 714 are out of focus with respect to the emitter detector pair 716. The pumping apparatus detects rotation of the disk 708 and monitors operation of the pumping mechanism as described above. It is contemplated that the outer peripheral surface 710 may have reflective portions 712 while the inner peripheral surface 718 has non-reflective portions 714 and the emitter detector pair 716 may be focused on the outer peripheral surface 710.

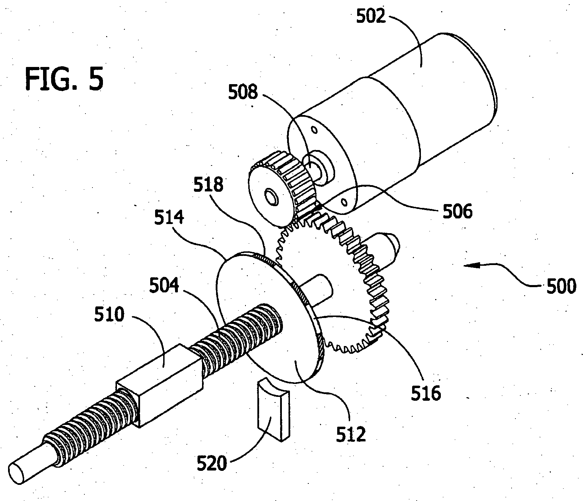

It is to be understood that the reflective and non-reflective portions shown in FIGS. 5, 6A, 6B, and 7 may be formed in a manner similar to reflective portions 212 and non-reflective portions 214 as described above.

When introducing elements of aspects of the invention or the embodiments thereof, the articles "a," "an," "the," and "said" are intended to mean that there are one or more of the elements. The terms "comprising," "including," and "having" are intended to be inclusive and mean that there may be additional elements other than the listed elements.

Having described aspects of the invention in detail, it will be apparent that modifications and variations are possible without departing from the scope of aspects of the invention as defined in the appended claims. As various changes could be made in the above constructions and products, without departing from the scope of aspects of the invention, it is intended that all matter contained in the above description and shown in the accompanying drawings shall be interpreted as illustrative and not in a limiting sense.

## Claims

1. A medical pumping apparatus (100) comprising:
a pump rotor (106) including a surface (210) having a reflective portion (212) for reflecting electromagnetic radiation and a non-reflective portion (214) that does not reflect electromagnetic radiation;
a motor (204) for rotating the pump rotor;
an emitter (208) for emitting electromagnetic radiation, said emitter positioned to emit electromagnetic radiation sequentially on the reflective and non-reflective portions of the surface of the pump rotor as the pump rotor rotates; and
a detector (208) for receiving electromagnetic radiation reflected by the surface of the pump rotor and providing a detection signal indicative of the received electromagnetic radiation for monitoring rotation of the pump rotor,
a reservoir,
a housing (104),
a pump set (102) supplying fluid from the reservoir to a patient, wherein the pump set (102) is loaded onto the housing (104) such that the pump set (102) is in contact with the pump rotor (106),
wherein when the pumping apparatus (100) rotates the pump rotor (106), rollers (108) occlude a portion of the pump set (102) and force the fluid from the reservoir to the patient by a peristaltic action;
**characterized in that**
the reflective portion (212) of the surface of the pump rotor comprises a smooth portion of the pump rotor produced when forming the pump rotor;
that the non-reflective portion (214) of the surface of the pump rotor is at least one of the following: hatched, scratched, pitted, and roughed, such that said non-reflective surface diffuses electromagnetic radiation incident thereon; and
that the pump rotor (106) is injection molded using a mold that has smooth portions which correspond to the reflective portions (212), and a rough portion corresponding to the non-reflective portion (214), or
that the pump rotor (106) is machined from a block of material such that the pump rotor (106) has smooth portions corresponding to the reflective portions (212),
and a rough portion corresponding to the non-reflective portion (214), or
that the pump rotor (106) is formed such that the entire surface of the pump rotor (106) is reflective with respect to electromagnetic radiation and wherein the non-reflective portion (214) is added by machining the surface of the pump rotor (106).

2. The medical pumping apparatus of claim 1 further comprising an output circuit (218) for receiving the detection signal and providing an output signal in response thereto, said output signal indicating whether the detector is receiving a predetermined amount of electromagnetic radiation reflected by the surface of the pump rotor.

3. The medical pumping apparatus of claim 1 or 2 wherein the emitter (208) and detector (208) are separated from the pump rotor (106) by a window (304), said window being transmissive with respect to the electromagnetic radiation.

4. The medical pumping apparatus of claim 1, 2 or 3 wherein:
the emitter emits infrared radiation; and
the surface of the pump rotor has a plurality of reflective portions (212) each separated by a non-reflective portion (214), said reflective portions being equally spaced from each other and equally spaced from an axis about which the pump rotor (106) rotates.

5. The medical pumping apparatus of one of the claims 1 to 4 wherein the motor (204) comprises a shaft (216) adapted to engage the pump rotor (106) to transfer rotational force from the motor to the pump rotor, and wherein said shaft extends perpendicularly from the surface (210) of the pump rotor along an axis of rotation about which the pump rotor rotates when engaged therewith.

6. A pump rotor (106) for a medical pumping apparatus (100), said medical pumping apparatus having a motor (204), an emitter (208), and a detector (208), a reservoir, a housing (104),
a pump set (102) supplying fluid from the reservoir to a patient, wherein the pump set (102) is loaded onto the housing (104) such that the pump set (102) is in contact with the pump rotor (106),
said pump rotor comprising:
a receiver constructed and arranged for engaging a motor shaft (216), said motor shaft defining an axis of rotation and supporting the pump rotor (106) when engaged by the receiver; and
a surface transverse (210) to the axis of rotation, said surface comprising a reflective portion (212) for reflecting electromagnetic radiation and a non-reflective portion (214) that does not reflect electromagnetic radiation, wherein:
the motor rotates the pump rotor via the shaft;
wherein when the pumping apparatus (100) rotates the pump rotor (106), rollers (108) occlude a portion of the pump set (102) and force the fluid from the reservoir to the patient by a peristaltic action;
the emitter (208) emits electromagnetic radiation onto the surface (210) of the pump rotor, said emitter being positioned to sequentially emit electromagnetic radiation on the reflective and non-reflective portions of the surface of the pump rotor as the pump rotor rotates about the axis of rotation; and
the detector (208) is positioned to receive electromagnetic radiation reflected by the surface (210) of the pump rotor;
**characterized in that**
the reflective portion (212) of the surface of the pump rotor comprises a smooth portion of the pump rotor produced when forming the pump rotor; and
that the non-reflective portion (214) of the surface of the pump rotor is at least one of the following: hatched, scratched, pitted, and roughed, such that said non-reflective surface diffuses electromagnetic radiation incident thereon; and
that the pump rotor (106) is injection molded using a mold that has smooth portions which correspond to the reflective portions (212), and a rough portion corresponding to the non-reflective portion (214), or that the pump rotor (106) is machined from a block of material such that the pump rotor (106) has smooth portions corresponding to the reflective portions (212), and a rough portion corresponding to the non-reflective portion (214), or
that the pump rotor (106) is formed such that the entire surface of the pump rotor (106) is reflective with respect to electromagnetic radiation and wherein the non-reflective portion (214) is added by machining the surface of the pump rotor (106).

7. The pump rotor of claim 6 wherein the reflective portion (212) of the surface of the pump rotor is constructed and arranged for reflecting infrared radiation emitted by the emitter (208).

8. The pump rotor of claim 6 or 7 wherein the surface of the pump rotor has a plurality of reflective portions (212) each separated by a non-reflective portion (214), said reflective portions being equally spaced from each other and equally spaced from the axis of rotation.

## Patentansprüche

1. Medizinische Pumpvorrichtung (100), die aufweist:
einen Pumpenrotor (106), der eine Oberfläche (210) mit einem reflektierenden Abschnitt (212) zum Reflektieren elektromagnetischer Strahlung und einem nicht reflektierenden Abschnitt (214), der elektromagnetische Strahlung nicht reflektiert, hat;
einen Motor (204) zum Drehen des Pumpenrotors;
einen Strahler (208) zum Emittieren elektromagnetischer Strahlung, wobei der Strahler positioniert ist, um elektromagnetische Strahlung nacheinander auf die reflektierenden und nicht reflektierenden Abschnitte der Oberfläche des Pumpenrotors zu emittieren,
während der Pumpenrotor sich dreht; und
eine Erfassungseinrichtung (208) zum Empfangen elektromagnetischer Strahlung, die von der Oberfläche des Pumpenrotors reflektiert wird, und Bereitstellen eines Erfassungssignals, das für die empfangene elektromagnetische Strahlung bezeichnend ist,
ein Reservoir,
eine Gehäuse (104),
ein Pumpenset (102), welches Flüssigkeit von dem Reservoir zu einem Patienten fördert, wobei das Pumpenset (102) derart auf das Gehäuse aufgesetzt ist, dass das Pumpenset mit dem Pumpenrotor (106) in Kontakt steht,
wobei, wenn die medizinische Pumpenvorrichtung (100) den Pumpenrotor (106) dreht, Rollen (108) einen Abschnitt des Pumpensets (102) verschließen und Flüssigkeit mittels eines peristaltischen Vorgangs von dem Reservoir zu dem Patienten treiben,
**dadurch gekennzeichnet,**
**dass** der reflektierende Abschnitt (212) der Oberfläche des Pumpenrotors einen glatten Abschnitt des Pumpenrotors aufweist, der hergestellt wird, wenn der Pumpenrotor ausgebildet wird;
**dass** der nicht reflektierende Abschnitt (214) der Oberfläche des Pumpenrotors wenigstens eines der folgenden ist: schraffiert, zerkratzt, körnig oder aufgeraut, so dass die nicht reflektierende Oberfläche darauf einfallende elektromagnetische Strahlung streut, und
**dass** der Pumpenrotor (106) im Spritzgussverfahren mit einer Form hergestellt ist, welche glatte Abschnitte, welche den reflektierenden Abschnitten (212) entsprechen, und einen rauen Abschnitt, welcher dem nicht reflektierenden Abschnitt (214) entspricht, aufweisen, oder
**dass** der Pumpenrotor (106) durch maschinelle Bearbeitung aus einem Block Material gefertigt ist, so dass der der Pumpenrotor (106) glatte Abschnitte, welche den reflektierenden Abschnitten (212) entsprechen, und einen rauen Abschnitt, welcher dem nicht reflektierenden Abschnitt (214) entspricht, aufweist, oder
**dass** der Pumpenrotor (106) derart gefertigt ist, dass die gesamte Oberfläche des Pumpenrotors (106) reflektierend gegenüber elektromagnetischer Strahlung ist und wobei der nicht reflektierende Abschnitt (214) durch maschinelle Bearbeitung der Oberfläche des Pumpenrotors (106) erzeugt ist.

2. Medizinische Pumpvorrichtung nach Anspruch 1, die ferner eine Ausgangsschaltung (218) aufweist, um das Erfassungssignal zu empfangen und ansprechend darauf ein Ausgangssignal bereitzustellen, wobei das Ausgangssignal anzeigt, ob die Erfassungseinrichtung eine vorgegebene Menge elektromagnetischer Strahlung, die von der Oberfläche des Pumpenrotors reflektiert wird, empfängt.

3. Medizinische Pumpvorrichtung nach Anspruch 1 oder 2, wobei der Strahler (208) und die Erfassungseinrichtung (208) durch ein Fenster (304) von dem Pumpenrotor (106) getrennt sind, wobei das Fenster in Bezug auf die elektromagnetische Strahlung durchlässig ist.

4. Medizinische Pumpvorrichtung nach Anspruch 1, 2 oder 3, wobei:
der Strahler Infrarotstrahlung emittiert; und
die Oberfläche des Pumpenrotors eine Vielzahl reflektierender Abschnitte (212) hat, von denen jeder durch einen nicht reflektierenden Abschnitt (214) getrennt ist, wobei die reflektierenden Abschnitte gleich voneinander beabstandet sind und gleich von einer Achse beabstandet sind, um die sich der Pumpenrotor (106) dreht.

5. Medizinische Pumpvorrichtung nach einem der Ansprüche 1 bis 4, wobei der Motor (204) eine Welle (216) aufweist, die geeignet ist, in den Pumpenrotor (106) einzugreifen, um Drehkraft von dem Motor an den Pumpenrotor zu übertragen, und wobei die Welle sich von der Oberfläche (210) des Pumpenrotors senkrecht entlang einer Drehachse erstreckt, um die der Pumpenrotor sich dreht, wenn sie mit ihm in Eingriff ist.

6. Pumpenrotor (106) für medizinische Pumpvorrichtung (100), wobei die medizinische Pumpvorrichtung einen Motor (204), einen Strahler (208) und eine Erfassungseinrichtung, ein Reservoir, ein Gehäuse, ein Pumpenset, wobei das Pumpenset Flüssigkeit von dem Reservoir zu einem Patienten fördert, wobei das Pumpenset (102) derart auf das Gehäuse aufgesetzt ist, dass das Pumpenset mit dem Pumpenrotor (106) in Kontakt steht, hat,
wobei der Pumpenrotor aufweist:
einen Aufnehmer, die aufgebaut und eingerichtet ist, um in eine Motorwelle (216) einzugreifen, wobei die Motorwelle eine Drehachse definiert und den Pumpenrotor (106) hält, wenn von dem Aufnehmer in sie eingegriffen wird;
eine Oberfläche (210) quer zu der Drehachse, wobei die Oberfläche einen reflektierenden Abschnitt (212) zum Reflektieren elektromagnetischer Strahlung und einen nicht reflektierenden Abschnitt (214), der elektromagnetische Strahlung nicht reflektiert,
aufweist;
wobei
der Motor den Pumpenrotor über die Welle dreht;
wobei, wenn die medizinische Pumpenvorrichtung (100) den Pumpenrotor (106) dreht, Rollen (108) einen Abschnitt des Pumpensets (102) verschließen und Flüssigkeit mittels eines peristaltischen Vorgangs von dem Reservoir zu dem Patienten treiben,
der Strahler (208) elektromagnetische Strahlung auf die Oberfläche (210) des Pumpenrotors emittiert, wobei der Strahler positioniert ist, um nacheinander elektromagnetische Strahlung auf die reflektierenden und nicht reflektierenden Abschnitte der Oberfläche des Pumpenrotors zu emittieren, während der Pumpenrotor sich um die Drehachse dreht;
die Erfassungseinrichtung (208) positioniert ist, um elektromagnetische Strahlung zu empfangen, die von der Oberfläche des Pumpenrotors (210) reflektiert wird;
**dadurch gekennzeichnet,**
der reflektierende Abschnitt (212) der Oberfläche des Pumpenrotors einen glatten Abschnitt des Pumpenrotors aufweist, der hergestellt wird, wenn der Pumpenrotor ausgebildet wird;
dass der nicht reflektierende Abschnitt (214) der Oberfläche des Pumpenrotors wenigstens eines der folgenden ist: schraffiert, zerkratzt, körnig oder aufgeraut, so dass die nicht reflektierende Oberfläche darauf einfallende elektromagnetische Strahlung streut,
dass der Pumpenrotor (106) im Spritzgussverfahren mit einer Form hergestellt ist, welche glatte Abschnitte, welche den reflektierenden Abschnitten (212) entsprechen, und einen rauen Abschnitt, welcher dem nicht reflektierenden Abschnitt (214) entspricht, aufweisen, oder
dass der Pumpenrotor (106) durch maschinelle Bearbeitung aus einem Block Material gefertigt ist, so dass der der Pumpenrotor (106) glatte Abschnitte, welche den reflektierenden Abschnitten (212) entsprechen, und einen rauen Abschnitt, welcher dem nicht reflektierenden Abschnitt (214) entspricht, aufweist, oder
dass der Pumpenrotor (106) derart gefertigt ist, dass die gesamte Oberfläche des Pumpenrotors (106) reflektierend gegenüber elektromagnetischer Strahlung ist und wobei der nicht reflektierende Abschnitt (214) durch maschinelle Bearbeitung der Oberfläche des Pumpenrotors (106) erzeugt ist.

7. Pumpenrotor nach Anspruch 6, wobei der reflektierende Abschnitt (212) der Oberfläche des Pumpenrotors aufgebaut und angeordnet ist, um Infrarotstrahlung zu reflektieren, die von dem Strahler (208) emittiert wird.

8. Pumpenrotor nach Anspruch 6 oder 7, wobei die Oberfläche des Pumpenrotors eine Vielzahl von reflektierenden Abschnitten (212) hat, die jeweils durch einen nicht reflektierenden Abschnitt (214) getrennt sind, wobei die reflektierenden Abschnitte gleich voneinander beabstandet und gleich von der Drehachse beabstandet sind.

## Revendications

1. Appareil de pompage médical (100) comprenant:
un rotor de pompe (106) comportant une surface (210) ayant une portion réfléchissante (212) pour réfléchir un rayonnement électromagnétique et une portion non-réfléchissante (214) qui ne réfléchit pas le rayonnement électromagnétique;
un moteur (204) pour faire tourner le rotor de pompe;
un émetteur (208) pour émettre un rayonnement électromagnétique, ledit émetteur étant positionné pour émettre un rayonnement électromagnétique séquentiellement sur les portions réfléchissante et non-réfléchissante de la surface du rotor de pompe lorsque le rotor de pompe tourne; et
un détecteur (208) pour recevoir le rayonnement électromagnétique réfléchi par la surface du rotor de pompe et fournissant un signal de détection indicatif du rayonnement électromagnétique reçu pour surveiller la rotation du rotor de pompe,
un réservoir,
un boitier (104)
un dispositif de pompage (102) fournissant du fluide du réservoir à un patient, où le dispositif de pompage (102) est logé dans le boitier (104) de sorte que le dispositif de pompage (102) est en contact avec le rotor de pompe (106),
où quand l'appareil de pompage (100) entraine en rotation le rotor de pompe (106), des rouleaux (108) occluent une portion du dispositif de pompage (102) et forcent le fluide à circuler du réservoir au patient par action péristaltique ;
**caractérisé en ce que**
la portion réfléchissante (212) de la surface du rotor de pompe comprend une portion lisse du rotor de pompe produite lors de la formation du rotor de pompe ;
**en ce que** la portion non réfléchissante (214) de la surface du rotor de pompe est au moins une des suivantes: hâchurée, rayée, piquée et rendue rugueuse, de sorte que ladite surface non réfléchissante diffuse le rayonnement électromagnétique incident sur celle-ci ; et
**en ce que** le rotor de pompe (106) est moulé par injection par utilisation d'un moule qui a des parties lisses correspondant aux portions réfléchissantes (212), et une portion rugueuse correspondant à la portion non réfléchissante (214), ou que le rotor de pompe (106) est usiné à partir d'un bloc de matériau de sorte que le rotor de pompe (106) possède des portions lisses correspondant aux portions réfléchissantes (212), et un portion rugueuse correspondant à la portion non réfléchissante (214), ou
**en ce que** le rotor de pompe (106) est formé de sorte que la totalité de la surface du rotor de pompe (106) est réfléchissant relativement au rayonnement électromagnétique et où la portion non réfléchissante (214) est ajoutée par usinage de la surface du rotor de pompe (106).

2. Appareil de pompage médical selon la revendication 1, comprenant en outre un circuit de sortie (218) pour recevoir le signal de détection et pour fournir un signal de sortie en réponse à celui-ci, ledit signal de sortie indiquant lorsque le détecteur reçoit une quantité prédéterminée de rayonnement électromagnétique réfléchi par la surface du rotor de pompe.

3. Appareil de pompage médical selon la revendication 1 ou 2, dans lequel l'émetteur (208) et le détecteur (208) sont séparés du rotor de pompe (106) par une fenêtre (304), ladite fenêtre étant transmissive relativement au rayonnement électromagnétique.

4. Appareil de pompage médical selon la revendication 1, 2 ou 3, dans lequel:
l'émetteur émet un rayonnement infrarouge; et
la surface du rotor de pompe possède une pluralité de portions de réflexion (212), chacune séparée par une portion non-réfléchissante (214), lesdites portions réfléchissantes étant également espacées les unes des autres et également espacées d'un axe autour duquel le rotor de pompe (106) tourne.

5. Appareil de pompage médical selon l'une des revendications 1 à 4, dans lequel le moteur (204) comprend un arbre (216) apte à venir en prise avec le rotor de pompe (106) pour transférer la force de rotation du moteur au rotor de pompe, et où ledit arbre s'étend perpendiculairement de la surface (210) du rotor de pompe le long d'un axe de rotation autour duquel le rotor de pompe tourne lorsqu'il est en prise avec celui-ci.

6. Rotor de pompe (106) pour un appareil de pompage médical (100), ledit appareil de pompage médical ayant un moteur (204), un émetteur (208) et un détecteur (208), un réservoir, un boitier (104),
un dispositif de pompage (102) fournissant du fluide du réservoir à un patient, où le dispositif de pompage (102) est logé dans le boitier (104) de sorte que le dispositif de pompage (102) est en contact avec le rotor de pompe (106), ledit rotor de pompe comprenant:
un récepteur construit et agencé pour venir en prise avec un arbre de moteur (216), ledit arbre de moteur définissant un axe de rotation et supportant le rotor de pompe (106) lorsque le récepteur est en prise avec celui-ci; et
une surface transversale (210) à l'axe de rotation, ladite surface comprenant une portion réfléchissante (212) pour réfléchir le rayonnement électromagnétique et une portion non-réfléchissante (214) qui ne réfléchit pas le rayonnement électromagnétique, où :
le moteur fait tourner le rotor de pompe par l'arbre;
où quand l'appareil de pompage (100) entraine en rotation le rotor de pompe (106), des rouleaux (108) occluent une portion du dispositif de pompage (102) et forcent le fluide à circuler du réservoir au patient par action péristaltique ;
l'émetteur (208) émet un rayonnement électromagnétique sur la surface (210) du rotor de pompe, ledit émetteur étant positionné pour émettre séquentiellement le rayonnement électromagnétique sur les portions réfléchissante et non-réfléchissante de la surface du rotor de pompe lorsque le rotor de pompe tourne autour de l'axe de rotation; et
le détecteur (208) est positionné pour recevoir le rayonnement électromagnétique réfléchi par la surface (210) du rotor de pompe;
**caractérisé en ce que**
la portion réfléchissante (212) de la surface de rotor de pompe comprend une portion lisse du rotor de pompe produite lors de la formation du rotor de pompe; et **en ce que** la portion non-réfléchissante (214) de la surface du rotor de pompe est au moins une des suivantes: hachurée, rayée, piquée et rendue rugueuse de sorte que ladite surface non-réfléchissante diffuse le rayonnement électromagnétique incident sur celle-ci ; et
en que le rotor de pompe (106) est moulé par injection par utilisation d'un moule qui a des parties lisses correspondant aux portions réfléchissantes (212), et une portion rugueuse correspondant à la portion non réfléchissante (214), ou que le rotor de pompe (106) est usiné à partir d'un bloc de matériau de sorte que le rotor de pompe (106) possède des portions lisses correspondant aux portions réfléchissantes (212), et un portion rugueuse correspondant à la portion non réfléchissante (214), ou
**en ce que** le rotor de pompe (106) est formé de sorte que la totalité de la surface du rotor de pompe (106) est réfléchissant relativement au rayonnement électromagnétique et où la portion non réfléchissante (214) est ajoutée par usinage de la surface du rotor de pompe (106).

7. Rotor de pompe selon la revendication 6, dans lequel la portion réfléchissante (212) de la surface du rotor de pompe est construite et agencée pour réfléchir le rayonnement infrarouge émis par l'émetteur (208).

8. Rotor de pompe selon la revendication 6 ou 7, dans lequel la surface du rotor de pompe possède une pluralité de portions de réflexion (212), chacune séparée par une portion non-réfléchissante (214), lesdites portions réfléchissantes étant uniformément espacées les unes des autres et uniformément espacées de l'axe de rotation.
